# EUROPEAN PATENT APPLICATION

(11) **EP 4 501 326 A1**
(43) Date of publication of application: **05.02.2025**
(21) Application number: 23780984.3
(22) Date of filing: 30.03.2023
(51) Int. Cl.: A61K 31/423, A61P 11/00, C07D 263/58

(54) **THERAPEUTIC AGENT FOR RESPIRATORY DISEASE**

(30) Priority: 31.03.2022 JP 2022059884
(71) Applicant: The Jikei University, Tokyo 105-8461 (JP); KOWA COMPANY, LTD., Naka-ku Nagoya-shi Aichi 460-8625 (JP)
(72) Inventor: ARAYA, Jun, Tokyo 105-8461 (JP); KUWANO, Kazuyoshi, Tokyo 105-8461 (JP); ITO, Saburo, Tokyo 105-8461 (JP); MATSUBAYASHI, Sachi, Tokyo 105-8461 (JP)
(74) Representative: Wächtershäuser & Hartz Patentanwaltspartnerschaft mbB
(86) International application number: PCT/JP2023/013377
(87) International publication number: WO 2023/190976

(57) **Abstract**

A novel therapeutic agent useful in treating a chronic pulmonary disease, such as a COPD or IPF. A pharmaceutical composition for treating a chronic pulmonary disease, including a therapeutically effective dose of (R)-2-[3-[[N-(benzoxazol-2-yl)-N-3-(4-methoxyphenoxy)propyl]aminomethyl]phenoxy]butyric acid or a salt thereof, or a solvate thereof.

## Description

### Technical Field

The present invention relates to treatment of a chronic pulmonary disease, such as a chronic obstructive pulmonary disease or idiopathic pulmonary fibrosis.

### Background Art

In recent years, an increase in morbidity of a chronic pulmonary disease, in particular, a chronic obstructive pulmonary disease (COPD) in elderly people has been problematic. The COPD has been pathophysiologically defined as follows: "irreversible airflow obstruction caused by an abnormal inflammation response to a damaging substance is progressively observed." Lesions involved in the pathogenesis of the COPD include a central airway lesion causing the hypersecretion of an airway, a peripheral airway lesion causing fixed airway obstruction, and an alveolar emphysema lesion. Particularly in a peripheral airway or a lung parenchyma, as the symptom of such lesion becomes serious, tissue disruption or fibrogenesis due to cell death is observed, and hence the symptom is largely different from the symptom of bronchial asthma. It is apoptosis that contributes to the tissue destruction, and in particular, neutrophil infiltration and an excessive increase in amount of an elastase accompanying the infiltration may play a large role in the apoptosis.

Onset factors of a COPD include smoking as the most important one, and hence emphasis has been placed on smoking cessation guidance as an effective prevention method in terms of everyday clinical practice. Other factors include a genetic factor, airway hyperreactivity, lung growth, exposure to a damaging substance in a workplace, the inhalation of a pollutant inside or outside a room, and infection. For a pharmacotherapy of the COPD, under the present circumstances, general therapeutic agents for asthma, such as a beta agonist, an anticholinergic drug, a xanthine preparation, and a steroid drug, have been applied per se. However, the effect of the administration of such drugs is to alleviate a symptom induced by the COPD, and hence suppression of the progression of a lesion or alleviation of the lesion cannot be expected. Accordingly, the development of a drug that can suppress the abnormalization of a lung tissue involved in a COPD patient has been strongly desired.

As a result of recent investigations, it has been reported that senescence and autophagy are each involved in the origin of a chronic pulmonary disease, such as a COPD or idiopathic pulmonary fibrosis (IPF) (Non Patent Literatures 1 to 3), and hence the establishment of a novel therapeutic method based on their control has been expected.

Transcription factor EB (TFEB) has been known as a transcription factor that controls the autophagy or lysosome biosynthesis, and its expression is regulated by PPARα (Non Patent Literatures 4 and 5). In fact, it has been reported that gemfibrozil known as a PPARα agonist exhibits an effect on a COPD through TFEB control (Non Patent Literatures 6 and 7).

Patent Literature 1 discloses that a compound represented by the following formula (1), a salt thereof, or a solvate thereof has a selective PPARα-activating action, and is hence useful as a preventive and/or therapeutic agent for hyperlipidemia, arteriosclerosis, diabetes, a diabetic complication (e.g., diabetic nephropathy), inflammation, a heart disease, or the like that is not accompanied by a weight gain or obesity in a mammal including a human: 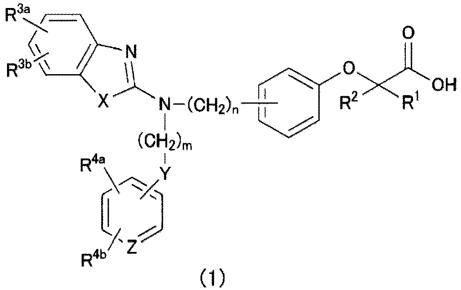 where R¹ and R² may be identical to or different from each other, and each represent a hydrogen atom, a methyl group, or an ethyl group, R^{3a}, R^{3b}, R^{4a}, and R^{4b} may be identical to or different from each other, and each represent a hydrogen atom, a halogen atom, a nitro group, a hydroxy group, a C₁₋₄ alkyl group, a trifluoromethyl group, a C₁₋₄ alkoxy group, a C₁₋₄ alkylcarbonyloxy group, a di-C₁₋₄ alkylamino group, a C₁₋₄ alkylsulfonyloxy group, a C₁₋₄ alkylsulfonyl group, a C₁₋₄ alkylsulfinyl group, or a C₁₋₄ alkylthio group, or R^{3a} and R^{3b}, or R^{4a} and R^{4b} are bonded to each other to represent an alkylenedioxy group, X represents an oxygen atom, a sulfur atom, or N-R⁵ (R⁵ represents a hydrogen atom, a C₁₋₄ alkyl group, a C₁₋₄ alkylsulfonyl group, or a C₁₋₄ alkyloxycarbonyl group), Y represents an oxygen atom, a S(O)₁ group ("1" represents a number of from 0 to 2), a carbonyl group, a carbonylamino group, an aminocarbonyl group, a sulfonylamino group, an aminosulfonyl group, or an NH group, Z represents CH or N, "n" represents a number of from 1 to 6, and "m" represents a number of from 2 to 6.

However, there is no description or suggestion of what action such a compound has on a chronic pulmonary disease, such as a COPD or IPF.

### Citation List

### Patent Literature

[PTL 1] WO 2005/023777 A1

### Non Patent Literatures

[NPL 1] Kuwano K, et al.: Respir Invest (2016) 54: 397
[NPL 2] Hosaka Y, et al.: Inflamm Regener (2021) 41: 29
[NPL 3] Liao S-X, et al.: Ther Adv Respir Dis (2019) 13: 1
[NPL 4] Ghosh A and Pahan K: Pharmacol Res (2016) 103: 144.
[NPL 5] Settembre C, et al.: Nat Cell Biol (2013) 15: 647.
[NPL 6] Bodas M, et al.: Antiox Redox Signal (2017) 27(3): 150
[NPL 7] Bodas M, et al.: Free Rad Biol Med (2019) 131: 81

### Summary of Invention

### Technical Problem

An object of the present invention is to provide a novel therapeutic agent useful in treating a chronic pulmonary disease, such as a COPD or IPF.

### Solution to Problem

To achieve the above-mentioned object, the present inventors made energetic investigations, and as a result, found that quite surprisingly, a compound disclosed as Example 85 in Patent Literature 1 described above, that is, (R)-2-[3-[[N-(benzoxazol-2-yl)-N-3-(4-methoxyphenoxy)propyl]aminomethyl]phenoxy]butyric acid (hereinafter may be referred to as "pemafibrate") was useful in treating a chronic pulmonary disease, such as a COPD or IPF. In this way, the present inventors completed the present invention.

That is, the present invention provides the following [1] to [15].
[1] A pharmaceutical composition for treating a chronic pulmonary disease, comprising a therapeutically effective dose of (R)-2-[3-[[N-(benzoxazol-2-yl)-N-3-(4-methoxyphenoxy)propyl]aminomethyl]phenoxy]butyric acid or a salt thereof, or a solvate thereof.
[2] The pharmaceutical composition according to [1], wherein the chronic pulmonary disease is a chronic obstructive pulmonary disease.
[3] The pharmaceutical composition according to [1], wherein the chronic pulmonary disease is idiopathic pulmonary fibrosis.
[4] A therapeutic agent for a chronic pulmonary disease, comprising, as an active ingredient, (R)-2-[3-[[N-(benzoxazol-2-yl)-N-3-(4-methoxyphenoxy)propyl]aminomethyl]phenoxy]butyric acid or a salt thereof, or a solvate thereof.
[5] The therapeutic agent according to [4], wherein the chronic pulmonary disease is a chronic obstructive pulmonary disease.
[6] The therapeutic agent according to [4], wherein the chronic pulmonary disease is idiopathic pulmonary fibrosis.
[7] A method for treating a chronic pulmonary disease, comprising administering (R)-2-[3-[[N-(benzoxazol-2-yl)-N-3-(4-methoxyphenoxy)propyl]aminomethyl]phenoxy]butyric acid or a salt thereof, or a solvate thereof to a patient in need thereof.
[8] The method according to [7], wherein the chronic pulmonary disease is a chronic obstructive pulmonary disease.
[9] The method according to [7], wherein the chronic pulmonary disease is idiopathic pulmonary fibrosis.
[10] (R)-2-[3-[[N-(Benzoxazol-2-yl)-N-3-(4-methoxyphenoxy)propyl]aminomethyl]phenoxy]butyric acid or a salt thereof, or a solvate thereof for use in treatment of a chronic pulmonary disease.
[11] The (R)-2-[3-[[N-(benzoxazol-2-yl)-N-3-(4-methoxyphenoxy)propyl]aminomethyl]phenoxy]butyric acid or a salt thereof, or a solvate thereof according to [10], wherein the chronic pulmonary disease is a chronic obstructive pulmonary disease.
[12] The (R)-2-[3-[[N-(benzoxazol-2-yl)-N-3-(4-methoxyphenoxy)propyl]aminomethyl]phenoxy]butyric acid or a salt thereof, or a solvate thereof according to [10], wherein the chronic pulmonary disease is idiopathic pulmonary fibrosis.
[13] Use of (R)-2-[3-[[N-(benzoxazol-2-yl)-N-3-(4-methoxyphenoxy)propyl]aminomethyl]phenoxy]butyric acid or a salt thereof, or a solvate thereof for production of a pharmaceutical composition for treating a chronic pulmonary disease.
[14] The use according to [13], wherein the chronic pulmonary disease is a chronic obstructive pulmonary disease.
[15] The use according to [13], wherein the chronic pulmonary disease is idiopathic pulmonary fibrosis.

### Advantageous Effects of Invention

The present invention provides a novel therapeutic agent useful in treating a chronic pulmonary disease, such as a COPD or IPF. The present invention facilitates to provide a novel therapeutic alternative to a patient of the chronic pulmonary disease, such as the COPD or IPF, in whom an effect exhibited by a conventional therapeutic agent is not sufficiently observed, and a patient of the chronic pulmonary disease, such as the COPD or IPF, in whom it is difficult to use the current therapeutic agent.

### Brief Description of Drawings

FIG. 1 shows an action of pemafibrate on the induction of TEFB expression in a human airway epithelial cell.
FIG. 2 shows an action of pemafibrate on smoking stimulation-induced cell senescence.
FIG. 3 shows an action of pemafibrate on the induction of TEFB expression in a cigarette smoke-exposed COPD model.
FIG. 4 shows an action of pemafibrate on a cellular distribution in a bronchoalveolar lavage fluid in the cigarette smoke-exposed COPD model.
FIG. 5 shows a tissue image of an action of pemafibrate on pulmonary emphysema in the cigarette smoke-exposed COPD model.
FIG. 6 shows an action of pemafibrate on pulmonary emphysema in the cigarette smoke-exposed COPD model.
FIG. 7 shows an action of pemafibrate on cell senescence in the cigarette smoke-exposed COPD model.
FIG. 8 shows an action of pemafibrate in a bleomycin pulmonary fibrosis model.

### Description of Embodiments

(R)-2-[3-[[N-(Benzoxazol-2-yl)-N-3-(4-methoxyphenoxy)propyl]aminomethyl]phenoxy]butyric acid (pemafibrate) to be used in the present invention is represented by the following chemical formula (A):

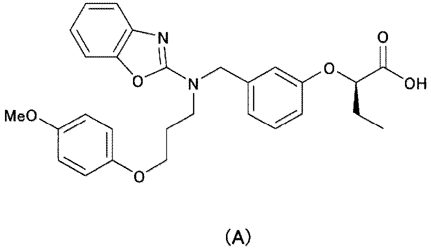

The compound may be produced in accordance with, for example, a method described in WO 2005/023777 A1. The compound may be formulated to a preparation in accordance with a method described in a literature. Further, a preparation containing pemafibrate is approved as a therapeutic agent for hyperlipidemia "PARMODIA (registered trademark) TABLET" in Japan, and the "PARMODIA TABLET" may be used.

In one embodiment of the present invention, a salt or a solvate of pemafibrate may be used. The salt and the solvate may each be produced by an ordinary method. While the salt of pemafibrate is not particularly limited as long as the salt is pharmaceutically acceptable, examples thereof include: alkali metal salts, such as a sodium salt and a potassium salt; alkaline earth metal salts, such as a calcium salt and a magnesium salt; organic base salts, such as an ammonium salt and a trialkylamine salt; mineral acid salts, such as a hydrochloride and a sulfate; and organic acid salts such as an acetate. Examples of the solvate of pemafibrate or of the salt thereof include a hydrate and an alcohol adduct (e.g., an ethanol adduct).

As described in Examples to be described later, pemafibrate increases the expression of TFEB in lungs of a mouse chronic obstructive pulmonary disease (COPD) model, and suppresses the destruction of the structure of the lungs due to cigarette smoke exposure. In addition, pemafibrate suppresses an increase in concentration of a macrophage in a bronchoalveolar lavage fluid due to the cigarette smoke exposure, and suppresses cell senescence. Further, pemafibrate suppresses an increase in amount of hydroxyproline in lungs of a bleomycin-induced mouse pulmonary fibrosis model known as an animal model for idiopathic pulmonary fibrosis (IPF). Accordingly, in one aspect of the present invention, administration of a pharmaceutical composition comprising a therapeutically effective dose of pemafibrate or a salt thereof, or a solvate thereof to a patient of a chronic pulmonary disease, such as a COPD or IPF, serves to treating chronic pulmonary diseases, such as the COPD or IPF.

In the present invention, a chronic obstructive pulmonary disease (COPD) is defined as follows: "The COPD is a pulmonary disease caused by, for example, the inhalation of, or exposure to, a hazardous substance typified by cigarette smoke for a long period of time, and shows airflow obstruction in a respiratory function test. The airflow obstruction is caused by the complex involvement of a peripheral airway lesion and an emphysematous lesion at various ratios. The COPD clinically shows dyspnea on exertion, or a chronic cough or phlegm, which progresses gradually, but may be poor in such symptom." (edited by the production committee of the 5th edition of the COPD guideline of the Japanese Respiratory Society: Guideline for Diagnosis and Treatment of Chronic Obstructive Pulmonary Disease (COPD) 2018, 5th edition, 2018).

In addition, idiopathic pulmonary fibrosis (IPF) refers to one kind of "idiopathic interstitial pneumonia," and to "a poor-prognosis disease, which has a chronic and progressive course, and in which a high degree of fibrogenesis progresses to cause irreversible honeycomb lung formation" (Guideline for Diagnosis and Treatment of Idiopathic Interstitial Pneumonia: the production committee of the guideline for the diagnosis and treatment of diffuse pulmonary diseases of the Japanese Respiratory Society, Health Labor Sciences Research, specific disease countermeasure business, diffuse pulmonary disease research group).

The symptoms of those chronic pulmonary diseases include a dry cough (cough without phlegm), phlegm, shortness of breath, crepitus, and a clubbed finger, but are not limited to such symptoms.

A COPD and IPF are each diagnosed by, for example, a smoking history, a clinical symptom or a clinical sign, a blood test (e.g., an oxygen amount or a serum marker), a respiratory function test (e.g., a vital capacity), or an imaging diagnosis (e.g., a chest X-ray or high-resolution CT). A lung biopsy may be performed.

In the present invention, the treatment of a chronic pulmonary disease, such as a COPD or IPF, refers to one or more selected from the group consisting of: an improvement in respiratory function; the alleviation of the above-mentioned various symptoms; and the suppression of the progression of lung fibrogenesis.

In one aspect of the present invention, the pharmaceutical composition comprising pemafibrate or a salt thereof, or a solvate thereof may be formulated to preparations, such as a tablet, a capsule, a granule, a powder, a lotion, an ointment, an injection, an inhalant, or a suppository, through use of any other pharmaceutically acceptable carrier. Those preparations may each be produced by a known method.

Examples of the pharmaceutically acceptable carrier may include an excipient, a disintegrator, a binder, a lubricant, a plasticizer, a glidant, a diluent, a solubilizer, a suspending agent, an isotonizing agent, a pH adjuster, a buffer, a stabilizer, a coating agent, a colorant, a taste-masking agent, and an odor-masking agent.

In one aspect of the present invention, the pharmaceutical composition comprising pemafibrate or a salt thereof, or a solvate thereof is preferably in the form of an inhalant, from the viewpoint of targeting a chronic pulmonary disease. The inhalant is not particularly limited, and is appropriately selected from, for example, an inhalation powder, an inhalation liquid, and an inhalation aerosol. At the time of the use of the inhalant of the present invention, an appropriate instrument or device has only to be used for its inhalation and administration, or the inhalant has only to be loaded into a container comprising an instrument for inhalation.

In one aspect of the present invention, the therapeutically effective dose of pemafibrate or a salt thereof, or a solvate thereof, and the number of times of the administration thereof may be appropriately set by a person skilled in the art, though the dose and the number vary depending on, for example, the body weight, age, sex, and symptom of a patient. In the case of, for example, a normal adult, pemafibrate may be administered in a dose of from 0.05 mg to 0.8 mg per day in 1 to 3 portions, and is administered preferably in a dose of from 0.2 mg to 0.4 mg per day in 1 or 2 portions, more preferably in a dose of from 0.1 mg to 0.8 mg per day in 1 or 2 portions.

A subject to whom the pharmaceutical composition of the present invention is to be administered is, for example, a human in whom the treatment of a chronic pulmonary disease, such as a COPD or IPF, is desired or needed. A human in whom an effect exhibited by a current therapeutic agent is not sufficiently observed, or it is difficult to use the conventional therapeutic agent is preferred.

The present invention is more specifically described below by way of Examples. However, these Examples are not intended to limit the present invention.

### Examples

### Example 1: Induction of TEFB Expression in Human Airway Epithelial Cell

The effect of pemafibrate was examined with reference to a procedure described in a literature (Araya J, et al.: Autophagy (2019), 15(3): 510). That is, cultured human airway epithelial cells (BEAS2B) were cultured in a culture medium supplemented with 10 nM or 100 nM of pemafibrate for 24 hours, and were compared to a control (0 nM).

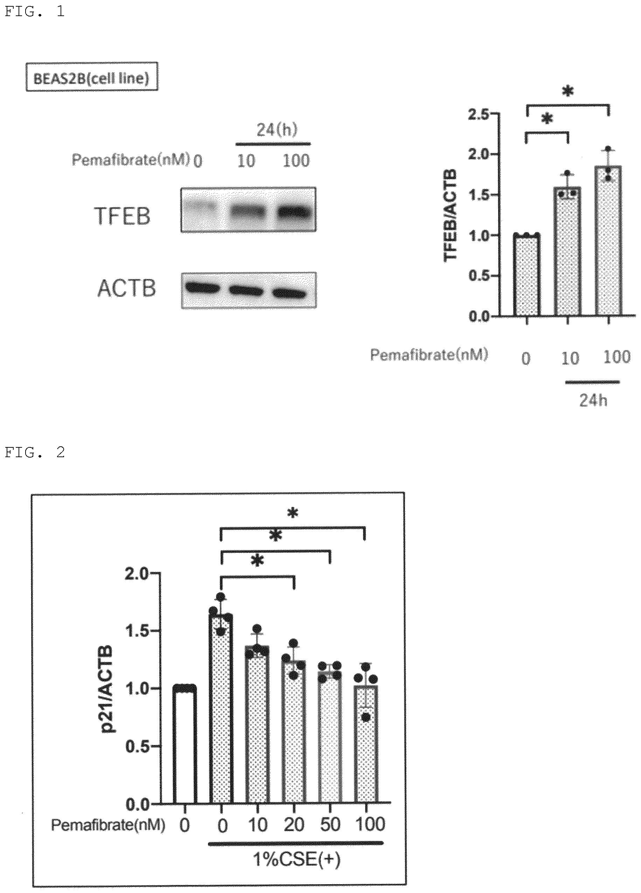

The results are shown in FIG. 1 (Mean±SE, n=3). It was recognized that the expression of TFEB increased in a manner dependent on the dose of pemafibrate. As the ratio of the expression amount of TFEB to the expression amount of ACTB serving as a reference was examined, a significant difference was observed as compared to the control (*: p<0.05, ANOVA and Turkey post-hoc test).

### Example 2: Action on Smoking Stimulation-induced Cell Senescence

The effect of pemafibrate was examined with reference to a procedure described in a literature (Saito N, et al.: J Immunol (2019), 202: 1428). That is, human-derived cultured airway epithelial cells (HBEC) were cultured in a culture medium supplemented with 10 nM to 100 nM of pemafibrate together with 1% cigarette smoke extract (CSE) for 24 hours, and were compared to a control (0 nM).

The results are shown in FIG. 2 (Mean±SE, n=4). The expression of p21 serving as an indicator of apoptosis was increased by CSE stimulation, but was suppressed by pemafibrate in a dose-dependent manner. As the ratio of the expression amount of p21 to the expression amount of ACTB serving as a reference was examined, a significant difference was observed at a pemafibrate concentration of 20 nM or more as compared to the control (*: p<0.05, ANOVA and Turkey post-hoc test). The results recognized that pemafibrate had a suppressing action on cell senescence.

### Example 3: Action on Cigarette Smoke-exposed COPD Model

The effect of pemafibrate was examined with reference to a procedure described in a literature (Araya J, et al.: Autophagy (2019), 15(3): 510). That is, C57BL/6J mice were exposed to cigarette smoke with an exposure and inhalation system for small animals five times a week for 6 months. The mice were classified into a total of 4 groups as follows: the mice were classified into a normal diet group (Ctrl) and a 0.0003 w/w% pemafibrate mixed feed group (Pema), and the groups were each further classified into a room air group (RA) and a cigarette smoke exposure group (CS).

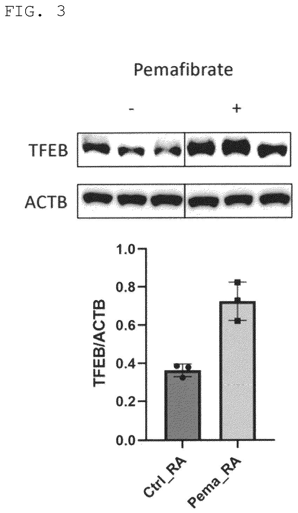

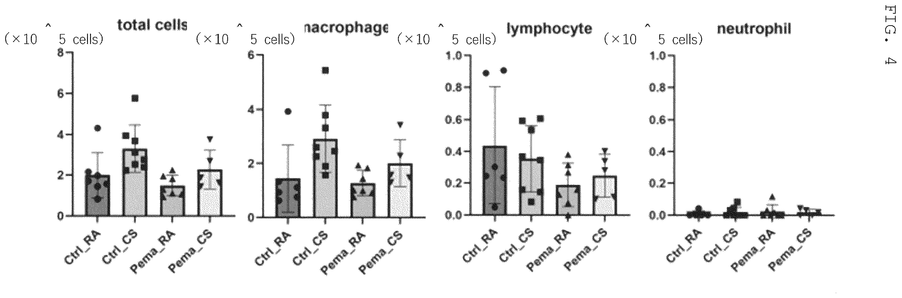

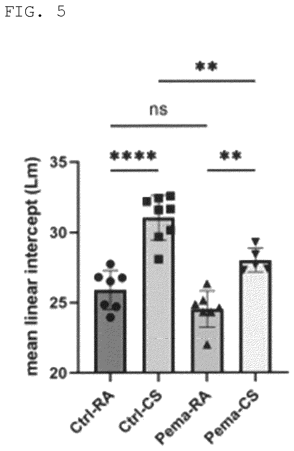

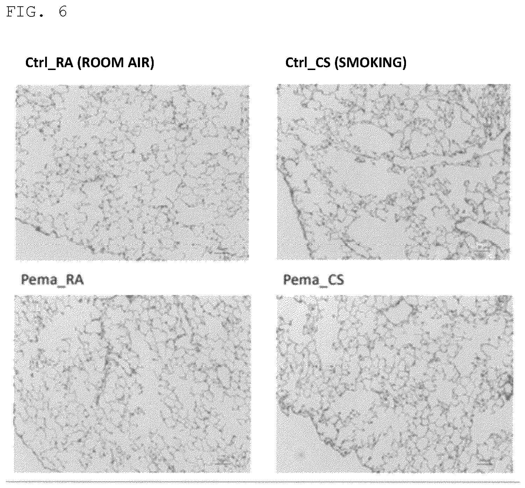

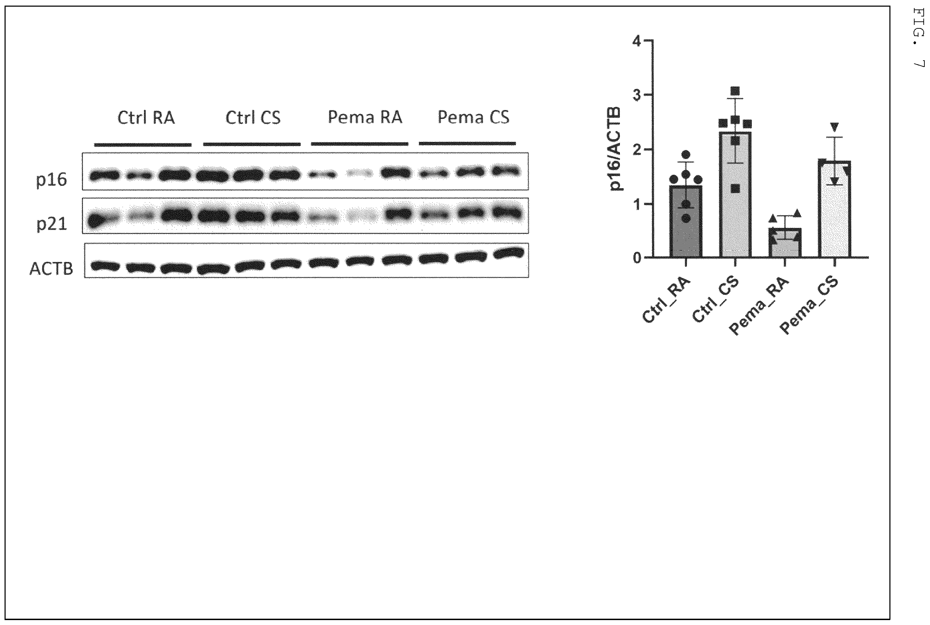

The results are shown in FIG. 3 to FIG. 7. First, a lung after the completion of the administration of pemafibrate was removed from the room air group (RA), and was evaluated for the expression of TFEB by being homogenized. As a result, an increase in expression of TFEB was observed in the pemafibrate-administered group (FIG. 3: Mean±SE, n=3). Next, a bronchoalveolar lavage fluid was collected and evaluated for its cells. As a result, it was revealed that a macrophage whose concentration had been increased by the cigarette smoke exposure (CS) was suppressed in the pemafibrate-administered group (FIG. 4: Mean±SE, n=5 to 8). In addition, an alveolar diameter was measured and the degree of emphysema was evaluated. As a result, it was found that the destruction of a lung structure due to the cigarette smoke exposure (CS) was suppressed in the pemafibrate-administered group (FIG. 5: Mean±SE, n=5 to 8 (**: p<0.01, ****: p<0.001, ANOVA and Turkey post-hoc test) and FIG. 6). Further, the evaluation of cell senescence revealed that the cell senescence due to the cigarette smoke exposure (CS) was suppressed in the pemafibrate-administered group (FIG. 7: Mean±SE, n=4 to 6).

### Example 4: Action on Bleomycin Pulmonary Fibrosis Model

The effect of pemafibrate was examined with reference to a procedure described in a literature (Kadota T, et al., J Extracell Vesicles. 2021 Aug; 10(10): e12124). That is, C57BL/6J mice were classified into a total of 4 groups in accordance with the presence or absence of the administration of bleomycin (BLM/NS), and the presence or absence of the administration of pemafibrate (Ctrl/Pema). In each of the BLM groups, bleomycin (2.5 U) was administered to induce fibrogenesis, and pemafibrate was administered as 0.0003 w/w% mixed feed on and after the 7th day after the administration of BLM. A lung was removed on the 21st day after the administration of BLM, and the amount of hydroxyproline in the lung was measured.

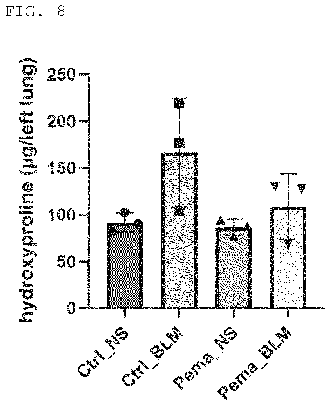

The results are shown in FIG. 8 (Mean±SE, n=3). It was confirmed that the amount of hydroxyproline serving as an indicator of the fibrogenesis was increased by the administration of bleomycin, but was suppressed by the administration of pemafibrate.

Thus, Examples 1 to 3 suggested that pemafibrate had a cell senescence-suppression action, and was hence useful in COPD treatment, and Example 4 suggested that pemafibrate was useful in IPF treatment.

### Industrial Applicability

The present invention was completed on the basis of the fact that the present inventors were the first to find the following, and the present invention is useful as a medicine for treating a chronic pulmonary disease: pemafibrate is recognized to have a therapeutic effect on a COPD or IPF.

## Claims

1. A pharmaceutical composition for treating a chronic pulmonary disease, comprising a therapeutically effective dose of (R)-2-[3-[[N-(benzoxazol-2-yl)-N-3-(4-methoxyphenoxy)propyl]aminomethyl]phenoxy]butyric acid or a salt thereof, or a solvate thereof.

2. The pharmaceutical composition according to claim 1, wherein the chronic pulmonary disease is a chronic obstructive pulmonary disease.

3. The pharmaceutical composition according to claim 1, wherein the chronic pulmonary disease is idiopathic pulmonary fibrosis.

4. A method for treating a chronic pulmonary disease, comprising administering an effective dose of (R)-2-[3-[[N-(benzoxazol-2-yl)-N-3-(4-methoxyphenoxy)propyl]aminomethyl]phenoxy]butyric acid or a salt thereof, or a solvate thereof to a patient in need thereof.

5. The method according to claim 4, wherein the chronic pulmonary disease is a chronic obstructive pulmonary disease.

6. The method according to claim 4, wherein the chronic pulmonary disease is idiopathic pulmonary fibrosis.

7. (R)-2-[3-[[N-(Benzoxazol-2-yl)-N-3-(4-methoxyphenoxy)propyl]aminomethyl]phenoxy]butyric acid or a salt thereof, or a solvate thereof for use in treatment of a chronic pulmonary disease.

8. The (R)-2-[3-[[N-(benzoxazol-2-yl)-N-3-(4-methoxyphenoxy)propyl]aminomethyl]phenoxy]butyric acid or a salt thereof, or a solvate thereof according to claim 7, wherein the chronic pulmonary disease is a chronic obstructive pulmonary disease.

9. The (R)-2-[3-[[N-(benzoxazol-2-yl)-N-3-(4-methoxyphenoxy)propyl]aminomethyl]phenoxy]butyric acid or a salt thereof, or a solvate thereof according to claim 7, wherein the chronic pulmonary disease is idiopathic pulmonary fibrosis.

10. Use of (R)-2-[3-[[N-(benzoxazol-2-yl)-N-3-(4-methoxyphenoxy)propyl]aminomethyl]phenoxy]butyric acid or a salt thereof, or a solvate thereof for production of a pharmaceutical composition for treating a chronic pulmonary disease.

11. The use according to claim 10, wherein the chronic pulmonary disease is a chronic obstructive pulmonary disease.

12. The use according to claim 10, wherein the chronic pulmonary disease is idiopathic pulmonary fibrosis.
